# EUROPEAN PATENT APPLICATION

(11) **EP 4 834 915 A2**
(43) Date of publication of application: **04.11.2026**
(21) Application number: 26166086.4
(22) Date of filing: 19.03.2026
(51) Int. Cl.: C07K 5/103, C07K 7/06, C07K 7/08, C07K 14/00, G01N 33/68

(54) **SYSTEM PERFORMANCE STANDARD FOR ANALYTICAL INSTRUMENTS**

(30) Priority: 01.05.2025 US 202563798104 P
(71) Applicant: Agilent Technologies, Inc., Santa Clara, CA 95051 (US)
(72) Inventor: HSIAO, Jordy, Santa Clara (US); KNIERMAN, Michael, Santa Clara (US)
(74) Representative: Grünecker Patent- und Rechtsanwälte PartG mbB

(57) **Abstract**

A peptide-based analytical standard mixture, e.g., for mass spectrometry, is disclosed. The peptide-based analytical standard mixture includes at least five distinct peptide sequences. A first subset of the peptide sequences are representative of post-translational modifications. A second subset of the peptide sequences include peptide sequences that form metal adducts. Methods of facilitating mass spectrometer performance testing by providing the peptide-based analytical standard mixture are disclosed. Kits for performing mass spectrometer performance testing including the peptide-based analytical standard mixture are also disclosed.

## Description

### SEQUENCE LISTING

A Sequence Listing conforming to the rules of WIPO Standard ST.26 is hereby incorporated by reference. Said Sequence Listing has been filed as an electronic document via PatentCenter encoded as XML in UTF-8 text. The electronic document, created on April 1, 2025, is entitled "A2208-702000_ST26.xml", and 14,930 bytes in size.

### SUMMARY OF THE INVENTION

In accordance with an aspect, there is provided a peptide-based analytical standard mixture. The mixture may include at least five distinct peptide sequences. A first subset of the peptide sequences may be representative of post-translational modifications. A second subset of the peptide sequences may include peptide sequences that form metal adducts.

In some embodiments, the first subset of the peptide sequences may include peptide sequences that can be oxidized or deamidated.

In certain embodiments, the first subset of the peptide sequences may include the peptide of SEQ ID NO.1.

In certain embodiments, the first subset of the peptide sequences may include the peptide of SEQ ID NO: 2.

In certain embodiments, the first subset of the peptide sequences may include the peptide of SEQ ID NO: 3.

In some embodiments, the second subset of the peptide sequences may include one or more peptides that can form metal adducts. For example, the one or more peptides that can form metal adducts may adduct with iron, cobalt, and/or nickel.

In certain embodiments, the second subset of the peptide sequences may include the peptide of SEQ ID NO: 4.

In certain embodiments, the second subset of the peptide sequences may include the peptide of SEQ ID NO: 5.

In further embodiments, the peptide-based analytical standard mixture may include a third subset of peptide sequences. The third set of peptide sequences may include sequences that replicate peptide sequences of light chains and heavy chains of monoclonal antibodies. For example, the third subset of peptide sequences may include peptide sequences that are not conserved, e.g., a region of the antibody protein or the corresponding DNA/RNA sequence that has significantly changed during evolution, in immunoglobulin G1 (IgG1). In other embodiments, the third subset of peptide sequences may include peptide sequences that are conserved, e.g., a region of the antibody protein or the corresponding DNA/RNA sequence that has remained relatively unchanged during evolution, in IgG1.

In certain embodiments, the third subset of the peptide sequences may include the peptide of SEQ ID NO: 6.

In certain embodiments, the third subset of the peptide sequences may include the peptide of SEQ ID NO: 7.

In certain embodiments, the third subset of the peptide sequences may include the peptide of SEQ ID NO: 8.

In certain embodiments, the third subset of the peptide sequences may include the peptide of SEQ ID NO: 9.

In certain embodiments, the third subset of the peptide sequences may include the peptide of SEQ ID NO: 10.

In further embodiments, the peptide-based analytical standard mixture may include a fourth subset of peptide sequences. The fourth subset of peptide sequences may include sequences having at least one residue that, upon detection, provides an assessment of a dynamic range in a mass spectrometer. For example, to assess dynamic range in the mass spectrometer, the fourth subset of peptide sequences may include peptides sequences having at least one residue with an isotopic substitution. The peptides sequences having at least one residue with an isotopic substitution in the peptide may be at a lysine residue. The lysine residue may be substituted at one or both of its carbon center or nitrogen center., e.g., isotopic substitution with one or both of ¹³C₆ or ¹⁵N₇.

In certain embodiments, the fourth subset of the peptide sequences may include the peptide of SEQ ID NO: 11.

In some embodiments, the peptide-based analytical standard mixture may include a fifth subset of peptide sequences. The fifth subset of peptide sequences may include sequences that are useful for assessing peak shape in a chromatogram. For example, the fifth subset of peptide sequences may include at least one sequence having a plurality of proline residues.

In certain embodiments, the fifth subset of the peptide sequences may include the peptide of SEQ ID NO: 12.

In some embodiments, the peptide-based analytical standard mixture may include a sixth subset of peptide sequences. The sixth subset of peptide sequences may include at least one sequence that is prone to fragmentation in an ion source of a mass spectrometer. For example, the sixth subset of peptide sequences may include at least one sequence derived from myoglobin.

In certain embodiments, the sixth subset of the peptide sequences may include the peptide of SEQ ID NO: 13.

In accordance with an aspect, there is provided a method of facilitating mass spectrometer, e.g., HPLC mass spectrometer, performance testing. The method may include providing a peptide-based analytical standard mixture. The peptide-based analytical standard mixture may include at least five distinct peptide sequences. A first subset of the peptide sequences in the peptide-based analytical standard mixture may be representative of post-translational modifications. A second subset of the peptide sequences in the peptide-based analytical standard mixture may include peptide sequences that form metal adducts. The method may include providing instructions to analyze the peptide-based analytical standard mixture using the mass spectrometer.

In some embodiments, the provided peptide-based analytical standard mixture may include a third subset of peptide sequences that replicate peptide sequences or a portion of peptide sequences of light chains and heavy chains of monoclonal antibodies.

In some embodiments, the provided peptide-based analytical standard mixture may include a fourth subset of peptide sequences having at least one residue that, upon detection, provides an assessment of a dynamic range in the mass spectrometer. For example, the fourth subset of peptide sequences may include matching sequences but at least one residue in the matching sequences has an isotopic substitution.

In some embodiments, the provided peptide-based analytical standard mixture may include a fifth subset of peptide sequences having at least one peptide sequence useful for assessing peak shape in a chromatogram.

In some embodiments, the provided peptide-based analytical standard mixture may include a sixth subset of peptide sequences having at least one peptide sequence prone to fragmentation in an ion source of the mass spectrometer.

In accordance with an aspect, there is provided a kit for performing mass spectrometer performance testing. The kit may include a peptide-based analytical standard mixture as disclosed herein, e.g., a peptide-based analytical standard mixture that may include at least five distinct peptide sequences, e.g., a peptide-based analytical standard mixture that may include a first subset of the peptide sequences are representative of post-translational modifications and a second subset of the peptide sequences comprise peptide sequences that form metal adducts.

### BRIEF DESCRIPTION OF THE DRAWINGS

The accompanying drawings are not intended to be drawn to scale. In the drawings, each identical or nearly identical component that is illustrated in various figures is represented by a like numeral. For purposes of clarity, not every component may be labeled in every drawing. In the drawings:
FIGS. 1A-1B illustrate liquid chromatograms for a peptide-based analytical standard mixture of this disclosure. FIG. 1A illustrates the chromatogram at a mobile phase temperature of 40 °C. FIG. 1B illustrates the chromatogram at a mobile phase temperature of 60 °C.
FIGS. 2A-2B illustrate liquid chromatograms and mass spectra for select peptides in the peptide-based analytical standard mixture of this disclosure. FIG. 2A illustrates chromatograms for metal adduct peptide species. FIG. 2B illustrates mass spectra for metal adduct peptide species.

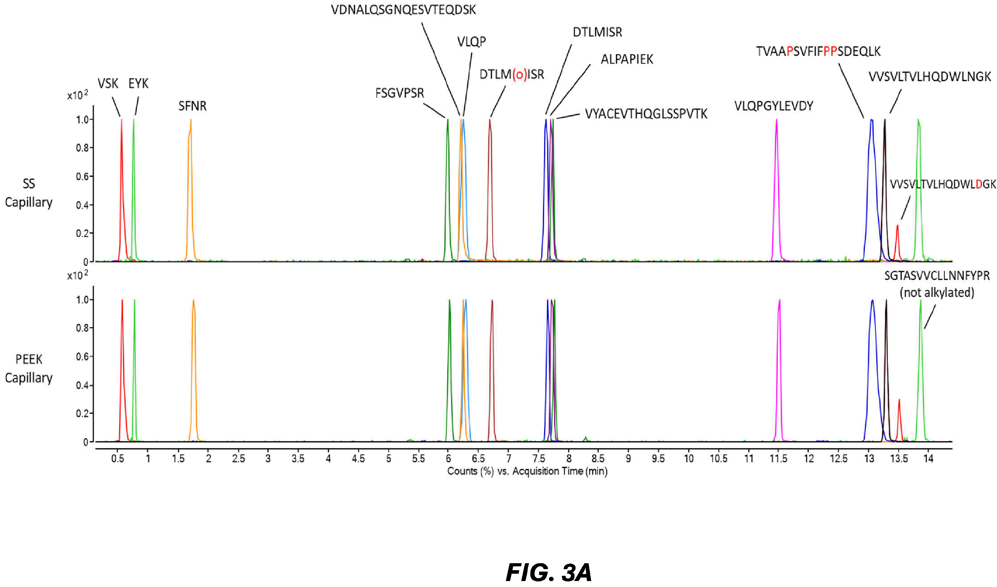

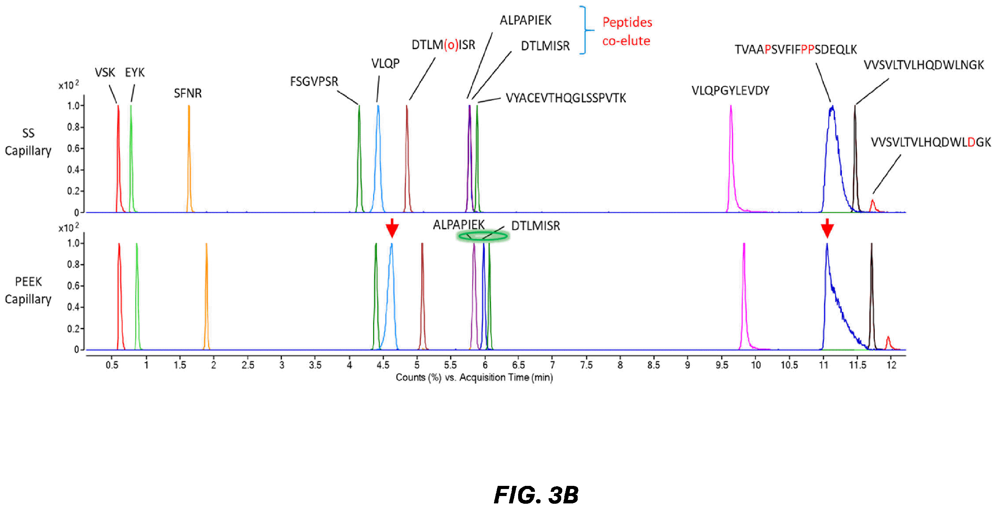

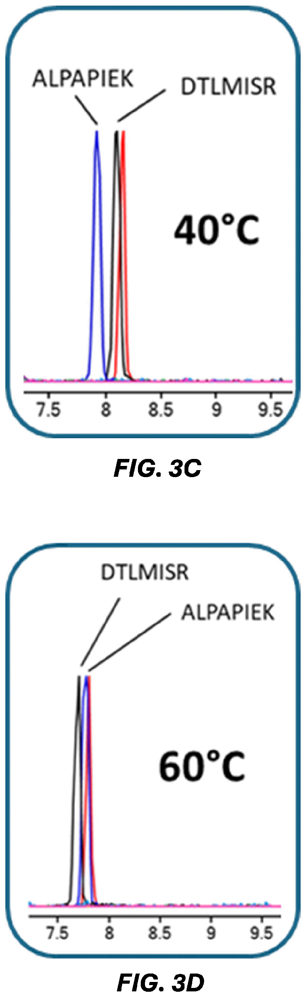

FIGS. 3A-3D illustrate liquid chromatograms for a peptide-based analytical standard mixture of this disclosure used for separation methodology troubleshooting. FIG. 3A illustrates liquid chromatograms for the peptide-based analytical standard mixture separated using two different capillary materials at 40 °C. FIG. 3B illustrates liquid chromatograms for the peptide-based analytical standard mixture separated using two different capillary materials at 60 °C. FIGS. 3C and 3D illustrate clipped liquid chromatograms showing previously measured elution orders for select peptides at 40 °C and 60 °C.

### DETAILED DESCRIPTION

This disclosure is not limited in its application to the details of construction and the arrangement of components set forth in the following description or illustrated in the drawings. The disclosure is capable of other embodiments and of being practiced or of being carried out in various ways. Also, the phraseology and terminology used herein is for the purpose of description and should not be regarded as limiting. The use of "including," "comprising," "having," "containing," "involving," and variations thereof herein is meant to encompass the items listed thereafter and equivalents thereof as well as additional items.

Mass spectrometry provides a rapid and sensitive technique for the determination of the molecular mass of a molecule or mixture of molecules. In the analysis of peptides and proteins, mass spectrometry can provide detailed information regarding, for example, the molecular mass, e.g., molecular weight or MW, of the original molecule, the molecular masses of peptides generated by proteolytic digestion of the original molecule, the molecular masses of fragments generated during the ionization of the original molecule, and even peptide sequence information for the original molecule and fragments thereof. Mass spectrometers are extremely precise; their performance and calibration must be carefully monitored as systematic errors may cause erroneous m/z values or changes in sensitivity.

Standard mixtures for the tuning and assessment of mass spectrometer performance are widely known. Typical standard mixtures are designed for specific applications, including but not limited to, replication of conserved sequences in monoclonal antibodies (mAbs), assessment of post-translational modification, e.g., oxidation and deamidation, assessment of peptide stability/fragility, characterization of peptide hydrophobicity/hydrophilicity, and mass spectrometer instrument performance, e.g., dynamic signal range, limit of detection/limit of quantification, and absolute quantification. Nearly all available standard mixtures lack the attributes that are of particular interest to the biopharmaceutical industry as they are incomplete and thus do not permit assessment of full LC/MS system performance. In addition to lacking one or more of the aforementioned features, many standard mixtures carry significant per-use costs, which limit implementation and thus effectiveness across the biopharmaceutical industry. It is an objective of this disclosure to provide an analytical standard mixture that provides for assessment of many biopharmaceutical parameters at an economic price point to enable routine use.

Disclosed herein are peptide-based analytical standard mixtures. The peptide-based analytical standard mixture includes at least five distinct peptide sequences. A first subset of the peptide sequences are representative of post-translational modifications. A second subset of the peptide sequences include peptide sequences that form metal adducts.

Post-translational modification are chemical modifications that play a key role in functional proteomics because they regulate activity, localization, and interaction with other cellular molecules such as proteins, nucleic acids, lipids and cofactors. Post-translational modifications are commonly employed to regulate cellular activity. Post-translational modifications occur at distinct amino acid side chains or peptide linkages, and they are most often mediated by enzymatic activity. Indeed, it is estimated that 5% of the proteome comprises enzymes that perform more than 200 types of post-translational modifications. These enzymes include kinases, phosphatases, transferases and ligases, which add or remove functional groups, proteins, lipids or sugars to or from amino acid side chains; and proteases, which cleave peptide bonds to remove specific sequences or regulatory subunits. Many proteins can also modify themselves using autocatalytic domains, such as autokinase and autoprotolytic domains. There are many types of post-translational modifications, including but not limited to, phosphorylation, hydroxylation, succinylation, oxidation, amidation, deamidation, formylation, glycosylation, and sulfation. In some embodiments, the first subset of the peptide sequences includes peptide sequences that can be oxidized. For example, a peptide that is prone to oxidation includes the peptide of SEQ ID NO: 1, i.e., Asp-Thr-Leu-Met-Ile-Ser-Arg, i.e., DTLMISR. One or more residues of the peptide of SEQ ID NO: 1 can be oxidized under suitable reaction conditions, Peptides that are prone to oxidation, e.g., the peptide of SEQ ID NO: 1, can be used to assess the performance of chromatography columns. Peptides prone to oxidation can be used to determine if an HPLC column has aged past the point of usability as the stationary phase material may oxidize the peptide. Peptides prone to oxidation can further be used to check for systematic defects in the HPLC column, e.g., poor peak shape, split peaks, the presence of shoulders or tails on peaks, lost resolution, reduced retention times, a drifted baseline, poor mobile phase preparation, and excessive back pressures.

In some embodiments, the first subset of the peptide sequences includes peptide sequences that can be deamidated. Deamidation is the hydrolysis of an amide functionality, resulting in the loss of ammonia. Peptide sequences that can be used to assess deamidation include the peptide of SEQ ID NO: 2, i.e., Val-Val-Ser-Val-Leu-Thr-Val-Leu-His-Gln-Asp-Trp-Leu-Asn-Gly-Lys, i.e., VVSVLTVLHQDWLNGK, and the peptide of SEQ ID NO: 3, i.e., Val-Val-Ser-Val-Leu-Thr-Val-Leu-His-Gln-Asp-Trp-Leu-Asp-Gly-Lys, i.e., VVSVLTVLHQDWLDGK. For example, the peptide of SEQ ID NO: 2 can be deamidated to hydrolyze the asparagine residue located at position 14 into aspartic acid, resulting in the peptide of SEQ ID NO: 3. Other post-translational modification, e.g., oxidation and deamidation, performed on the peptide sequences of the analytical standard mixture disclosed herein are within the scope of this disclosure.

Peptides that are capable of binding to metal atoms can be used to assess metal contamination in chromatography systems. For example, metal atoms can arise in a chromatography system from the degradation of certain components, e.g., columns, frits, tubing, fittings, and pumps. Metal atoms can further arise from poor preparation of buffer solutions. In use, peptides that can bind to metal atoms act as reporters for contamination based on a mass shift upon binding and analysis. In some embodiments, the second subset of the peptide sequences includes peptide sequences that can form metal adducts. Peptides of the second subset can form adducts with iron, cobalt and/or nickel. The peptides of the second subset can also form metal adducts with other transition metals.

In particular embodiments, the second subset of the peptide sequences includes the peptide of SEQ ID NO: 4, i.e., Val-Leu-Gln-Pro-Gly-Tyr-Leu-Glu-Val-Asp-Tyr, i.e., VLQPGYLEVDY.

In other embodiments, the second subset of the peptide sequences includes the peptide of SEQ ID NO: 5, i.e., Ala-Leu-Pro-Ala-Pro-Ile-Glu-Lys, i.e., ALPAPIEK.

Monoclonal antibodies (mAbs) based on immunoglobulins G (IgGs) are multichain glycoproteins with an approximate molecular weight of 150 kDa. They consist of four polypeptide chains: two light chains (Lc) of approximately 25 kDa each and two heavy chains (Hc) of approximately 50 kDa each, all linked together by disulfide bonds. MAbs are the fastest growing class of human therapeutics. The U.S. Food and Drug Administration has already approved over 80 therapeutic mAbs since 2018 and they are being used in the treatment of diseases related to cardiovascular, respiratory, hematology, kidney, immunology, and oncology systems. As with any other therapeutic, the molecular structure of mAbs must be well characterized to ensure drug safety, efficiency, batch-to-batch consistency, and stability over time.

Even the simplest mixture of two unique polypeptide chains obtainable via disulfide bond reduction without proteolysis, Lc and Hc, cannot be analyzed effectively by MS without some type of front-end fractionation that can isolate or partially separate the chains. Both the overlap of their charge state envelopes and ionization suppression effects can lower their spectral signal-to-noise ratio (S/N), particularly for the larger Hc, and could result in co-isolation during fragmentation experiments such as tandem MS. There exist few solutions in the marketplace that provide a standardized mixture of synthetic peptide sequences that are commonly found in mAbs, either conserved or non-conserved. In further embodiments, the peptide-based analytical standard mixture includes a third subset of peptide sequences that replicate peptide sequences of light chains and heavy chains of mAbs. The third subset of peptide sequences includes sequences that are conserved in immunoglobulin G1 and sequences that are not conserved in immunoglobulin G1.

In particular embodiments, the third subset of the peptide sequences includes the peptide of SEQ ID NO: 6, i.e., Val-Ser-Lys, i.e., VSK.

In particular embodiments, the third subset of the peptide sequences includes the peptide of SEQ ID NO: 7, i.e., Glu-Tyr-Lys., i.e., EYK.

In particular embodiments, the third subset of the peptide sequences includes the peptide of SEQ ID NO: 8, i.e., Ser-Phe-Asn-Arg, i.e., SFNR.

In particular embodiments, the third subset of the peptide sequences includes the peptide of SEQ ID NO: 9, i.e., Phe-Ser-Gly-Val-Pro-Ser-Arg, i.e., FSGVPSR.

In particular embodiments, the third subset of the peptide sequences includes the peptide of SEQ ID NO: 10, i.e., Val-Asp-Asn-Ala-Leu-Gln-Ser-Gly-Asn-Ser-Gln-Glu-Ser-Val-Thr-Glu-Gln-Asp-Ser-Lys, i.e., VDNALQSGNSQESVTEQDSK.

In mass spectrometry, the dynamic range describes the concentration range, in orders of magnitude, over which the instrument can provide a response that is proportional to the concentration of a particular compound. Functionally, the dynamic range can be defined as the ratio between the minimal and the maximal concentration of compounds that can be detected simultaneously in a given sample. The dynamic range depends on the sample complexity and on the type of mass spectrometer that is used. The benefit of having a wide linear dynamic range is especially important for small molecule quantitation during drug research, and for targeted peptide quantitation. In small molecule quantitation during drug research, the dosage form can cause an order of magnitude difference in quantitative results. In proteomics, the dynamic range of proteins in real samples can be in excess of 10 orders of magnitude with further complications arising from varying ionization efficiencies for the representative enzymatic peptide fragments. Thus, having a wide dynamic range makes experimentation simpler by avoiding re-assays and dilution issues. There exist few solutions in the marketplace that provide a standardized mixture of peptide sequences that can be used to assess the dynamic range of a mass spectrometer in a systematic fashion.

In further embodiments, the peptide-based analytical standard mixture includes a fourth subset of peptide sequences that have at least one residue that, upon detection, provides an assessment of the dynamic range of a mass spectrometer. This fourth set of peptide sequences generally includes two identical sequences by amino acid content where one or more residue in the peptide is isotopically labeled. The isotopic labeling of one or more residues provides for a consistent mass shift that is detectable by the mass spectrometer. All amino acids have a C-terminus with a free carboxylate group and an N-terminus with a free amino group. Further, every C atom and/or every N atom in an amino acid can be isotopically labeled to provide for a larger mass shift. Isotopic labeling for carbon atoms can be with carbon-13, i.e., labeled with ¹³C₆. Isotopic labeling for nitrogen atoms can be with nitrogen-15, i.e., labeled with ¹⁵N₇*.* In some embodiments, the fourth subset of peptide sequences includes at least one peptide sequence with an isotopically labeled lysine residue. The sequence with the isotopically labeled lysine residue includes labels at both the C-terminus and N-terminus of lysine, i.e., HO₂¹³C¹³CH(¹⁵NH₂)(¹³CH₂)₄¹⁵NH₃⁺, to provide for a +8 Da mass shift from standard lysine.

In particular embodiments, the fourth subset of the peptide sequences includes the peptide of SEQ ID NO: 11, i.e., Ala-Leu-Pro-Ala-Pro-Ile-Glu-Lys*, i.e., ALPAPIEK*, where Lys* or K* is isotopically labeled lysine at each carbon and nitrogen atom, i.e., HO₂¹³C^{l3}CH(¹⁵NH₂)(¹³CH₂)₄¹⁵NH₃.

A change in peak shape is one of the most common observations of problems with a liquid chromatography (LC) method. Because of this, may system suitability tests include a measure of peak shape such that a quantitative value of peak shape can be tracked over time. In chromatography applications, sharp peaks in chromatograms are preferred in because broad peaks or peaks with significantly distorted shapes, i.e., tailing, can easily overlap other species in the chromatogram, increasing the difficulty of interpreting samples. Poor peak shape can compromise the results of an analysis by degrading resolution between closely eluted peaks and reducing precision and accuracy of measuring peak area, especially for small peaks. The ideal chromatographic peak will have a Gaussian shape. Peptides that exhibit a change in peak shape as a function of a controllable variable, such as temperature, flow rate, and the like, can provide valuable information about the interactions between sample, mobile phase, and stationary phase in the column.

For example, the temperature of a component of a chromatography system, such as the column compartment, can have a direct impact on the peak shape of a species being eluted. When an analyte is injected into a liquid chromatography system, it enters the column in a narrow band. This narrow band of sample gradually becomes broader as the analytes in the sample interact with both the stationary phase and the mobile phase. The sample, under the proper conditions, should be eluted from the column with a Gaussian-shaped peak in a well-controlled liquid chromatography system. If the mobile phase is at ambient temperature, i.e., cold, and the column is heated to elevated temperatures, i.e., warm, the cooler mobile phase cools the inlet of the column, creating a temperature gradient in the column and peak distortion. In further embodiments, the peptide-based analytical standard mixture includes a fourth subset of peptide sequences having at least one peptide sequence useful for assessing peak shape in a chromatogram. The assessment of peak shape in a particular analyte is useful for diagnosing potential issues with one or more hardware components of a chromatography system, including but not limited to, pumps, columns, heat exchangers, ovens, and related hardware components. The assessment of peak shape in a particular analyte is further useful for diagnosing potential issues with separation methodologies, such as flow rate, pH, mobile phase composition, solvent gradients, sample injection volume, backpressure, and related parameters. In some embodiments, the fifth subset of peptide sequences includes at least one peptide sequence having a plurality of proline residues.

In particular embodiment, the fifth subset of peptide sequences comprise the peptide of SEQ ID NO: 12, i.e., Thr-Val-Ala-Ala-Pro-Ser-Val-Phe-Ile-Phe-Pro-Pro-Ser-Asp-Glu-Gln-Leu-Lys, i.e., TVAAPSVFIFPPSDEQLK.

In mass spectrometry, peptides and other larger molecules are ionized and then fragmented by collisions, e.g., collisions with inert gas molecules, non-reactive surfaces, or electrons. The resulting fragments ions are then analyzed to determine the peptide sequence. However, uncontrolled or poorly controlled fragmentation can lead to a number of issues with respect to protein or peptide analysis. Inaccurate or incomplete peptide sequences can lead to incorrect protein identification. Poor fragmentation, whether too much fragmentation or too little fragmentation, can make it difficult to identify the location of post-translational modifications on the peptide. Bad fragmentation can lead to a lower quality of proteomics data, making it difficult to draw meaningful conclusions from the analysis.

In particular, peptides that are prone to fragmentation in the ion source of a mass spectrometer can prove problematic for accurate quantitation in proteomics and other related mass spectrometry workups. Ion source fragmentation is directly related with the desolvation and activation energy at the interface of electrospray ionization (ESI) source. The desolvation/activation energy is controlled by the cone voltage, which is also called nozzle-skimmer voltage, fragmentor voltage, or declustering voltage. Higher desolvation/activation energy can enhance ion yield but simultaneously facilitate ion fragmentation due to elevated ion energy, which compromises the final ion yield. In practice, the ESI desolvation process is optimized by balancing these two inversely proportional factors to maximize ion gain and minimize fragmentation in the ion source to obtain accurate quantitation. Therefore, some degree of ion source fragmentation is an inherent phenomenon in ESI-MS, regardless of whether the resulting in-source fragments could be identified or not. There exist few solutions in the marketplace that provide a standardized mixture of peptide sequences that can be used to assess the extent of fragmentation of specific peptides in a mass spectrometer ion source in a systematic fashion, e.g., to explore the ionization parameter space of an ion source.

In further embodiments, the peptide-based analytical standard mixture includes a sixth subset of peptide sequences having at least one peptide sequence prone to fragmentation in an ion source of a mass spectrometer.

In particular embodiment, the fifth subset of peptide sequences comprise the peptide of SEQ ID NO: 13, i.e., Ala-Leu-Glu-Leu-Phe-Arg, i.e., ALELFR.

For any peptide sequence disclosed herein, the peptide sequence may include one or more substitutions to the given sequence. In general, substitution to peptide sequences do not alter the overall behavior of the peptide. Substitutions of residues are within the same or similar property class at the existing residue in the sequence. For example, aspartate (Asp, N) is an amino acid with a polar uncharged side chain and can be substituted with glutamine (Gln, Q). Lysine (Lys, K) has positively charged side chains and can be substituted with arginine (Arg, R). Serine (Ser, S), another amino acid with polar uncharged side chains, can be substituted with threonine (Thr, T) as it is similarly sized to serine and also has polar uncharged side chains. This disclosure is in no way limited by the type of substitutions for chemically similar amino acid residues in any of the peptide sequences disclosed herein.

In accordance with an aspect, there is provided a method of facilitating mass spectrometer performance testing. The method includes providing a peptide-based analytical standard mixture, e.g., a mixture as disclosed herein. The peptide-based analytical standard mixture includes at least five distinct peptide sequences. A first subset of the peptide sequences in the peptide-based analytical standard mixture are representative of post-translational modifications. A second subset of the peptide sequences in the peptide-based analytical standard mixture includes peptide sequences that form metal adducts. The method includes providing instructions to analyze the peptide-based analytical standard mixture using the mass spectrometer.

In some embodiments, the provided peptide-based analytical standard mixture includes a third subset of peptide sequences that replicate peptide sequences of light chains and heavy chains of monoclonal antibodies.

In some embodiments, the provided peptide-based analytical standard mixture includes a fourth subset of peptide sequences having at least one residue that, upon detection, provides an assessment of a dynamic range in the mass spectrometer.

In some embodiments, the provided peptide-based analytical standard mixture includes a fifth subset of peptide sequences having at least one peptide sequence useful for assessing peak shape in a chromatogram.

In some embodiments, the provided peptide-based analytical standard mixture includes a sixth subset of peptide sequences having at least one peptide sequence prone to fragmentation in an ion source of the mass spectrometer.

In accordance with an aspect, there is provided a kit for performing mass spectrometer performance testing. The kit includes a peptide-based analytical standard mixture as disclosed herein, e.g., a peptide-based analytical standard mixture that includes at least five distinct peptide sequences, e.g., a peptide-based analytical standard mixture that includes a first subset of the peptide sequences are representative of post-translational modifications and a second subset of the peptide sequences comprise peptide sequences that form metal adducts.

### EXAMPLES

The function and advantages of these and other embodiments can be better understood from the following examples. These examples are intended to be illustrative in nature and are not considered to be in any way limiting the scope of the invention.

### Example 1 - Temperature Dependence on Elution

In this example, the effects of temperature on the elution order of peptides in a peptide-based analytical standard mixture is described.

A peptide-based analytical standard mixture was used as the sample in an LC/MS experiment designed to text the effects of capillary and solvent temperature on separation. The LC/MS setup includes an Agilent 1290 Infinity binary pump connected to the inlet of an analytical column by a PEEK capillary. The outlet of the analytical column was connected to the entrance of an Agilent 6546 quadrupole time-of-flight (QToF) mass spectrometer. The separation of the peptides in a peptide-based analytical standard mixture was performed using a gradient separation with two mobile phases A and B. Mobile phase A included water and 0.1% formic acid. Mobile phase B included acetonitrile and 0.1% formic acid. The gradient used was 2-38% of mobile phase B for a 15 minute total run time. The mobile phase flow rate was 0.4 mL/min. The temperature of the column compartment was set to 40 °C or 60 °C. The sample injection volume was 5 µL for a peptide concentration of 5 pmol.

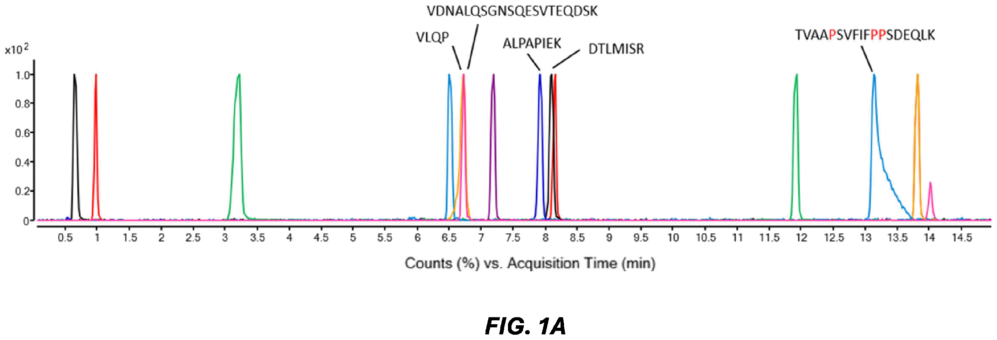

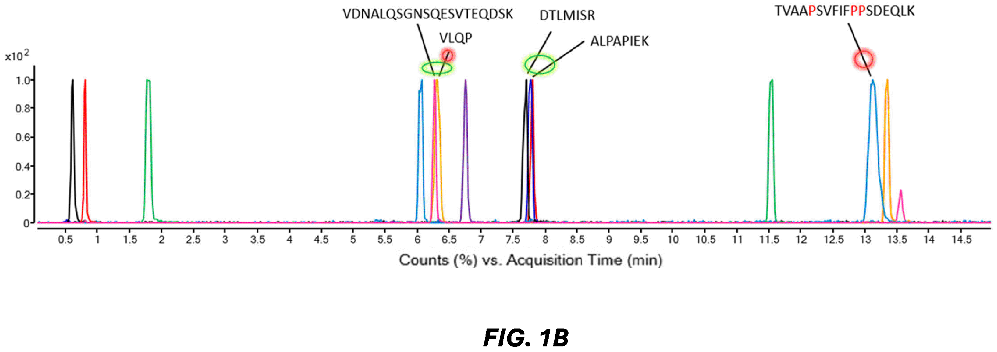

FIGS. 1A-1B illustrate the resulting normalized chromatograms with key peptide sequences labeled. FIG. 1A corresponds to the chromatogram with the mobile phase at 40 °C and FIG. 1B corresponds to the chromatogram with the mobile phase at 60 °C. There were two observations noted in the chromatograms of FIGS. 1A and 1B. First, the order of elution of peptides in the center of the run time changed when the column compartment was heated to 60 °C. At a column compartment temperature of 40 °C, VLQP eluted prior to VDNALQSGNSQESVTEQDSK and ALPAPIEK eluted prior to DTLMISR. At a column compartment temperature of 60 °C, the elution order was reversed for each pair, i.e., VDNALQSGNSQESVTEQDSK eluted prior to VLQP and DTLMISR eluted prior to ALPAPIEK. The second observation was the improvement in peak shape for the separation of the proline-rich peptide TVAAPSVFIFPPSDEQLK. At a column compartment temperature of 40 °C, the peak had a large tail. At a column compartment temperature of 60 °C, the peak shape substantially improved and exhibited less tailing.

### Example 2 - Determination of Iron Contamination in HLPC System

In this example, the use of peptides in a peptide-based analytical standard mixture to monitor trace metal contamination is described.

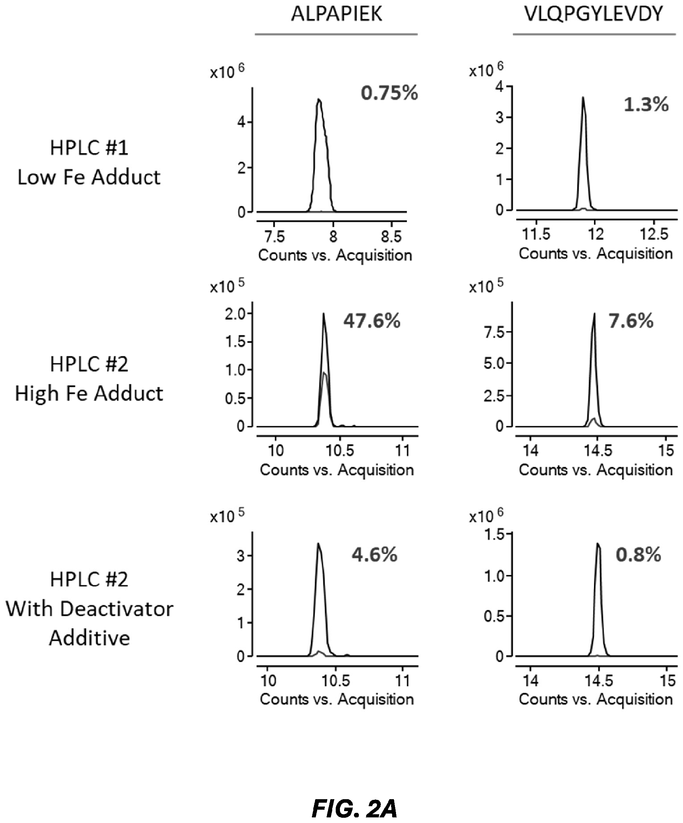

To demonstrate the utility of the peptide ALPAPIEK to monitor iron contamination in an HPLC system, the peptide mixture was injected onto two separate HPLC systems, labeled first and second. The injected mixture was subjected to LC/MS analysis on an Agilent 6545 Q-ToF instrument. The mass spectra are illustrated in FIGS. 2A-2B. The first set of mass spectra data (MS1) were acquired with the quadrupole mass analyzer. The masses of the unmodified and metal-adducted peptides were extracted to show the signal intensity differences between the two forms as illustrated in FIG. 2A. As illustrated in FIG. 2A, the first HPLC system yielded a low percentage of iron adducts at 0.75%. In contrast, the second HPLC system yielded an approximately 64x increase in iron adducts at 47.6%. These results showcased the ability of the peptide ALPAPIEK to differentiate the levels of metal contamination between different HPLC systems.

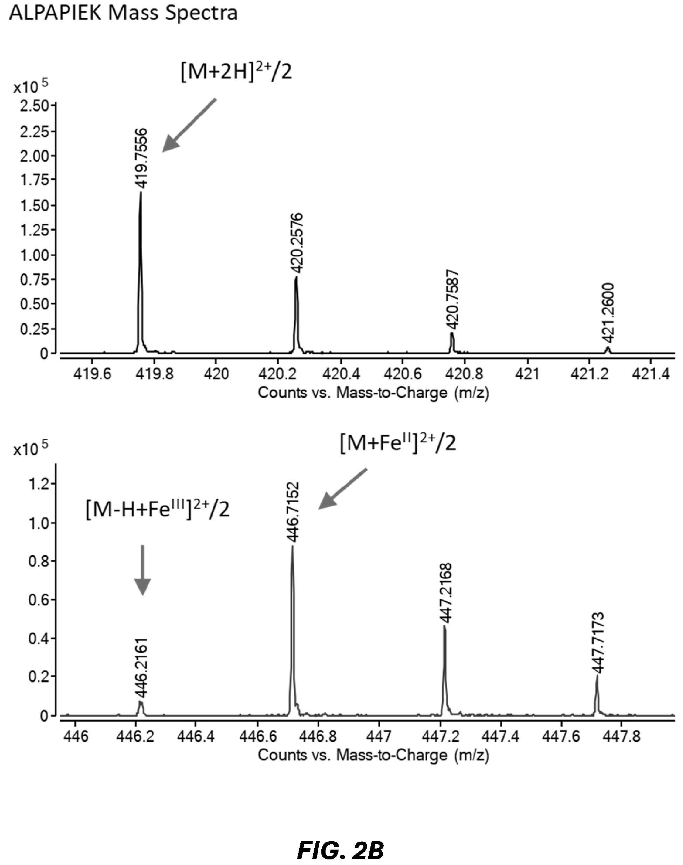

To confirm whether the observed masses were metal adducts, a metal-chelating additive, i.e., a deactivator additive, was introduced into the mobile phase solvents. Deactivator additives included pyrophosphoric acid or medronic acid, i.e., methylenediphosphonic acid. When the deactivator additive was added into the mobile phase solvents of the second HPLC system, the metal adducted form of the peptide reduced to less than 5%, which indicated removal of the metal from the second HPLC system. The mass spectra and the mono-isotopic masses were monitored using the ToF analyzer (MS2) for the unmodified and iron-adduct forms of the ALPAPIEK peptide are shown in FIG. 2B.

### Example 3 - Troubleshooting Separation Methods

In this example, the use of a peptide-based analytical standard mixture is described to troubleshoot existing separation methodologies is described.

A peptide-based analytical standard mixture was used as the sample in an LC/MS experiment designed to discover the reasons for errors in existing separation methods. The LC/MS setup includes an Agilent 1290 Infinity binary pump connected to the inlet of an analytical column by a PEEK capillary or a stainless steel (SS) capillary. The outlet of the analytical column was connected to the entrance of an Agilent 6546 quadrupole time-of-flight (QToF) mass spectrometer. The separation of the peptides in a peptide-based analytical standard mixture was performed using a gradient separation with two mobile phases A and B. Mobile phase A included water and 0.1% formic acid. Mobile phase B included acetonitrile and 0.1% formic acid. The gradient used was 2-38% of mobile phase B for a 15 minute total run time. The mobile phase flow rate was 0.4 mL/min and the mobile phase temperature was set to 40 °C or 60 °C. The sample injection volume was 5 µL for a peptide concentration of 5 pmol.

FIGS. 3A-3B illustrate the resulting normalized chromatograms with key peptide sequences labeled. FIG. 3A corresponds to the chromatogram with a SS capillary upstream of the analytical column and FIG. 3B corresponds to the chromatogram with a PEEK capillary upstream of the analytical column. In the chromatograms, the peptides ALPAPIEK and DTLMISR had different elution profile when using SS capillaries. With SS capillaries, these two peptides co-eluted from the column and when using PEEK capillaries, ALPAPIEK eluted prior to DTLMISR. This elution profile was unable to be reproduced on an independent and identical instrument setup in a different facility. It was noted that during previous acquisitions on the peptide mixture exploring the effects of mobile phase temperature in Example 1, ALPAPIEK eluted prior to DTLMISR with a mobile phase temperature of 40 °C and the elution order reversed with a mobile phase temperature of 60 °C. This is illustrated in FIGS. 3C and 3D. To discover the source of this error, the connections between the mobile phase containers and the column were verified. It was determined that when the PEEK capillary was used, the heat exchanger was bypassed. Thus, the mobile phase was not properly heated. This lack of proper mobile phase temperature was also verified with the poor peak shapes observed for the peptide VLQP, which showed fronting, and the proline-rich peptide TVAAPSVFIFPPSDEQLK showing substantial tailing over its elution window.

The phraseology and terminology used herein is for the purpose of description and should not be regarded as limiting. As used herein, the term "plurality" refers to two or more items or components. The terms "comprising," "including," "carrying," "having," "containing," and "involving," whether in the written description or the claims and the like, are open-ended terms, i.e., to mean "including but not limited to." Thus, the use of such terms is meant to encompass the items listed thereafter, and equivalents thereof, as well as additional items. Only the transitional phrases "consisting of" and "consisting essentially of," are closed or semi-closed transitional phrases, respectively, with respect to the claims. Use of ordinal terms such as "first," "second," "third," and the like in the claims to modify a claim element does not by itself connote any priority, precedence, or order of one claim element over another or the temporal order in which acts of a method are performed, but are used merely as labels to distinguish one claim element having a certain name from another element having a same name (but for use of the ordinal term) to distinguish the claim elements.

Having thus described several aspects of at least one embodiment, it is to be appreciated various alterations, modifications, and improvements will readily occur to those skilled in the art. Any feature described in any embodiment may be included in or substituted for any feature of any other embodiment. Such alterations, modifications, and improvements are intended to be part of this disclosure and are intended to be within the scope of the invention. Accordingly, the foregoing description and drawings are by way of example only.

Those skilled in the art should appreciate that the parameters and configurations described herein are exemplary and that actual parameters and/or configurations will depend on the specific application in which the disclosed methods and materials are used. Those skilled in the art should also recognize or be able to ascertain, using no more than routine experimentation, equivalents to the specific embodiments disclosed.
The invention is further embodied by the following items:
1. A peptide-based analytical standard mixture, comprising at least five distinct peptide sequences where a first subset of the peptide sequences are representative of post-translational modifications and a second subset of the peptide sequences comprise peptide sequences that form metal adducts.
2. The mixture of item 1, wherein the first subset of the peptide sequences includes peptide sequences that can be oxidized or deamidated.
3. The mixture of item 2, wherein the first subset of the peptide sequences comprises SEQ ID NO: 1, SEQ ID NO: 2, and SEQ ID NO: 3.
4. The mixture of item 1, wherein the second subset of the peptide sequences comprise peptides that can form metal adducts.
5. The mixture of item 4, wherein the second subset of the peptide sequences comprises SEQ ID NO: 4 and SEQ ID NO: 5.
6. The mixture of item 1, further comprising a third subset of peptide sequences that replicate peptide sequences of light chains and heavy chains of monoclonal antibodies.
7. The mixture of item 6, wherein the third subset of peptide sequences comprises peptide sequences that are not conserved in immunoglobulin G1.
8. The mixture of item 6, wherein the third subset of peptide sequences comprises peptide sequences that are conserved in immunoglobulin G1.
9. The mixture of item 6, wherein the third subset of peptide sequences comprises SEQ ID NO: 6, SEQ ID NO: 7, SEQ ID NO: 8, SEQ ID NO: 9, and SEQ ID NO: 10.
10. The mixture of item 6, comprising a fourth subset of peptide sequences that have at least one residue that, upon detection, provides an assessment of a dynamic range in a mass spectrometer.
11. The mixture of item 10, wherein the fourth subset of peptide sequences comprise peptides sequences having at least residue with an isotopic substitution.
12. The mixture of item 11, wherein the isotopic substitution comprises an isotopically labeled lysine residue.
13. The mixture of item 12, wherein the fourth subset of peptide sequences comprises the peptide of SEQ ID NO: 11.
14. The mixture of item 10, comprising a fifth subset of peptide sequences having at least one peptide sequence useful for assessing peak shape in a chromatogram.
15. The mixture of item 14, wherein the fifth subset of peptide sequences comprise at least one peptide sequence having a plurality of proline residues.
16. The mixture of item 15, wherein the fifth subset of peptide sequences comprise the peptide of SEQ ID NO: 12.
17. The mixture of item 14, comprising a sixth subset of peptide sequences having at least one peptide sequence prone to fragmentation in an ion source of a mass spectrometer.
18. The mixture of item 17, wherein the sixth subset of peptide sequences comprise peptides from myoglobin.
19. The mixture of item 18, wherein the sixth subset of peptide sequences comprises the peptide of SEQ ID NO: 13.
20. A method of facilitating mass spectrometer performance testing, comprising:
   providing a peptide-based analytical standard mixture, the peptide-based analytical standard mixture comprising at least five distinct peptide sequences where a first subset of the peptide sequences are representative of post-translational modifications and a second subset of the peptide sequences comprise peptide sequences that form metal adducts; and
   providing instructions to analyze the peptide-based analytical standard mixture using the mass spectrometer.
21. The method of item 20, wherein the provided peptide-based analytical standard mixture comprises a third subset of peptide sequences that replicate peptide sequences of light chains and heavy chains of monoclonal antibodies.
22. The method of item 21, wherein the provided peptide-based analytical standard mixture comprises a fourth subset of peptide sequences that have at least one residue that, upon detection, provides an assessment of a dynamic range in the mass spectrometer.
23. The method of item 22, wherein the provided peptide-based analytical standard mixture comprises a fifth subset of peptide sequences having at least one peptide sequence useful for assessing peak shape in a chromatogram.
24. The method of item 23, wherein the provided peptide-based analytical standard mixture comprises a sixth subset of peptide sequences having at least one peptide sequence prone to fragmentation in an ion source of the mass spectrometer.
25. A kit for performing mass spectrometer performance testing, comprising the peptide-based analytical standard mixture of item 1.

## Claims

1. A peptide-based analytical standard mixture, comprising at least five distinct peptide sequences where a first subset of the peptide sequences are representative of post-translational modifications and a second subset of the peptide sequences comprise peptide sequences that form metal adducts.

2. The mixture of claim 1, wherein the first subset of the peptide sequences includes peptide sequences that can be oxidized or deamidated.

3. The mixture of claim 2, wherein the first subset of the peptide sequences comprises SEQ ID NO: 1, SEQ ID NO: 2, and SEQ ID NO: 3.

4. The mixture of claim 1, wherein the second subset of the peptide sequences comprise peptides that can form metal adducts.

5. The mixture of claim 4, wherein the second subset of the peptide sequences comprises SEQ ID NO: 4 and SEQ ID NO: 5.

6. The mixture of claim 1, further comprising a third subset of peptide sequences that replicate peptide sequences of light chains and heavy chains of monoclonal antibodies.

7. The mixture of claim 6,
(i) wherein the third subset of peptide sequences comprises peptide sequences that are not conserved in immunoglobulin G1;
(ii) wherein the third subset of peptide sequences comprises peptide sequences that are conserved in immunoglobulin G1;
(iii) wherein the third subset of peptide sequences comprises SEQ ID NO: 6, SEQ ID NO: 7, SEQ ID NO: 8, SEQ ID NO: 9, and SEQ ID NO: 10; or
(iv) comprising a fourth subset of peptide sequences that have at least one residue that, upon detection, provides an assessment of a dynamic range in a mass spectrometer.

8. The mixture of claim 7(iv),
(i) wherein the fourth subset of peptide sequences comprise peptides sequences having at least residue with an isotopic substitution, wherein preferably the isotopic substitution comprises an isotopically labeled lysine residue, wherein more preferably the fourth subset of peptide sequences comprises the peptide of SEQ ID NO: 11; or
(ii) comprising a fifth subset of peptide sequences having at least one peptide sequence useful for assessing peak shape in a chromatogram.

9. The mixture of claim 8(ii),
(i) wherein the fifth subset of peptide sequences comprise at least one peptide sequence having a plurality of proline residues, wherein preferably the fifth subset of peptide sequences comprise the peptide of SEQ ID NO: 12; or
(ii) comprising a sixth subset of peptide sequences having at least one peptide sequence prone to fragmentation in an ion source of a mass spectrometer, wherein preferably the sixth subset of peptide sequences comprise peptides from myoglobin, wherein more preferably the sixth subset of peptide sequences comprises the peptide of SEQ ID NO: 13.

10. A method of facilitating mass spectrometer performance testing, comprising:
providing a peptide-based analytical standard mixture, the peptide-based analytical standard mixture comprising at least five distinct peptide sequences where a first subset of the peptide sequences are representative of post-translational modifications and a second subset of the peptide sequences comprise peptide sequences that form metal adducts; and
providing instructions to analyze the peptide-based analytical standard mixture using the mass spectrometer.

11. The method of claim 10, wherein the provided peptide-based analytical standard mixture comprises a third subset of peptide sequences that replicate peptide sequences of light chains and heavy chains of monoclonal antibodies.

12. The method of claim 11, wherein the provided peptide-based analytical standard mixture comprises a fourth subset of peptide sequences that have at least one residue that, upon detection, provides an assessment of a dynamic range in the mass spectrometer.

13. The method of claim 12, wherein the provided peptide-based analytical standard mixture comprises a fifth subset of peptide sequences having at least one peptide sequence useful for assessing peak shape in a chromatogram.

14. The method of claim 13, wherein the provided peptide-based analytical standard mixture comprises a sixth subset of peptide sequences having at least one peptide sequence prone to fragmentation in an ion source of the mass spectrometer.

15. A kit for performing mass spectrometer performance testing, comprising the peptide-based analytical standard mixture of claim 1.
